Europäisches Patentamt

European Patent Office

Office européen des brevets

(11) Veröffentlichungsnummer: **0 357 832**
**A1**

## EUROPÄISCHE PATENTANMELDUNG

(21) Anmeldenummer: **88114816.7**

(22) Anmeldetag: **09.09.88**

(51) Int. Cl.5 **C07C 29/76 , B01D 3/14 , C12F 1/08**

(43) Veröffentlichungstag der Anmeldung:
**14.03.90 Patentblatt 90/11**

(84) Benannte Vertragsstaaten:
**DE ES FR GB IT**

(71) Anmelder: **GEORG WESTPHAL ING. KG APPARATEBAU, VERFAHRENSTECHNIK Bahnhofstrasse 43 D-6050 Offenbach a.M.(DE)**

(72) Erfinder: **Westphal, Volker Edith-Stein-Strasse 30 D-6050 Offenbach/Main(DE)**
Erfinder: **Westphal, Stephan Bahnhofstrasse 43 D-6050 Offenbach/Main(DE)**

(74) Vertreter: **Schulze, Ilse, Dipl.-Chem. Gaisbergstrasse 3 D-6900 Heidelberg(DE)**

(54) **Kombiniertes Adsorptions-Rektifikationsverfahren zur Trennung eines Flüssigkeitsgemisches.**

(57) Es wird ein kombiniertes Adsorptions-Rektifikationsverfahren zur Trennung eines aus unterschiedlich siedenden Flüssigkeiten bestehenden Flüssigkeitsgemisches in eine höher siedende Flüssigkeit, insbesondere Wasser, und in eine tiefer siedende, insbesondere organische, Flüssigkeit mit einer Anfangskonzentration von mehr als 80 Vol.-%, durch Druckwechsel-Adsorption und Rektifikation beschrieben. Ein aus einem Verdampfer oder aus dem Kopf eines Verdampfers oder einer Rektifikationskolonne kommender Beschickungsstrom wird durch ein aus mindestens drei periodisch gesteuerten Adsorptionsbetten bestehendes Adsorptionssystem geleitet, dessen Adsorbentien vornehmlich die höher siedende Flüssigkeit adsorbieren. Die Adsorptionsbetten werden abwech-selnd regeneriert bzw. beladen und durch indirekte Heizung ständig über dem Taupunkt des zu trennenden Flüssigkeitsgemisches gefahren. Das abgetrennte leicht siedende reine Endprodukt wird in einem Kondensator kondensiert, der die unter Unterdruck betriebene Aufarbeitungskolonne, zur Aufarbeitung der Rückläufe aus den Festbettadsorbern, heizt.

EP 0 357 832 A1

EP 0 357 832 A1

## Kombiniertes Adsorptions-Rektifikationsverfahren zur Trennung eines Flüssigkeitsgemisches

Die Erfindung betrifft ein kombiniertes Adsorptions-Rektifikationsverfahren zur Trennung eines aus unterschiedlich siedenden Flüssigkeiten bestehenden Flüssigkeitsgemisches in eine höher siedende Flüssigkeit, insbesondere Wasser, und in eine tiefer siedende, insbesondere organische Flüssigkeit mit einer Anfangskonzentration von mehr als 80 Vol.-%, durch Druckwechsel-Adsorption und Rektifikation.

Als wirksamstes Verfahren zur Trennung eines Alkohol-Wassergemisches, beispielsweise Ethanol, hat sich die Rektifikation seit Jahrzehnten bestens bewährt. Allerdings ist die Trennung des bei 96 Vol.-% entstehenden Azeotropengemisches ohne großen apparativen und energetischen Aufwand mit keiner Rektifikation möglich. Ein herkömmliches und seit lange benutztes Verfahren ist die sogenannte "Schleppmittelrektifikation", die unter Zuhilfenahme von Schleppmitteln, wie Benzin, Benzol und dergleichen, durchgeführt wird. Ein solches Verfahren erfordert aber hohe Energiemengen, hohen Regel- und Apparateaufwand und stellt erhöhte Anforderungen an das Bedienungspersonal. Außerdem birgt das meist toxische Schleppmittel eine Verunreinigungsgefahr des reinen Ethanols, der oft in der Humanmedizin Anwendung finden soll.

Eine bereits weltweit eingesetzte Möglichkeit der Stofftrennung ist die Adsorption, und zwar sowohl Druck als auch Temperaturwechseladsorption. Sie wird seit Jahrzehnten unter anderem zur Lufttrocknung, Luftzerlegung, Gastrocknung, Lösungsmittelrückgewinnung, usw. eingesetzt und zeichnet sich durch relativ geringen Energie-, Apparate- und Regelaufwand aus. Sie ist jedoch nur zur Gastrennung sinnvoll einzusetzen, da flüssige Stoffe die wirksame Adsorptionskinetik entscheidend behindern.

Benutzt man deshalb die Adsorption zur Trennung von Flüssigkeiten, muß diese zuerst verdampft und darf während des Adsorptions- bzw. Desorptionsvorganges nie ihren vom Druck abhängigen Taupunkt unterschreiten. Erst nach Verlassen des Adsorbers darf die Flüssigkeit kondensiert werden, ohne den Adsorber auf Dauer zu belasten.

Sollen verhältnismäßig große Stoffmengen abgetrennt werden, kommt vorzugsweise die Druckwechseladsorption infrage, da die Temperaturwechseladsorption relativ lange Zyklen aufweist, was ein hohes Adsorbervolumen zwingend nötig machen würde. Bei jedem Druckwechsel kommt es jedoch zwangsläufig zu Druck- und Mengenschwankungen in der Speise- bzw. Ableitung der Adsorber. Diese Druck- und Mengenschwankungen übertragen sich zwangsläufig auf andere Anlagenteile, z.B. nach bzw. vor geschalteten Rektifikationen, Kühlkreisläufe, Vakuumpumpen, usw.. Es ist bekannt, daß solche Schwankungen äußerst problematisch auf eine stabil und kontinuierlich zu betreibende Rektifikation wirken.

Desweiteren treten bei der Regeneration (Desorption) Rückläufe aus den Adsorbern auf, die noch hohe Anteile Ethanol enthalten, die energetisch günstigst aufzuarbeiten sind. Zu dieser Ausarbeitung bietet sich die Rektifikation an, die wie bekannt, bis zum azeotropen Punkt die technisch sinnvollste Lösung ist. Soll diese Aufarbeitung in der den Adsorber speisenden Rektifikation geschehen, muß diese dampfförmige Flüssigkeit kondensiert oder verdichtet auf das Druckniveau der Rektifikation gebracht werden und im mittleren Teil dieser eingespeist werden. Diese zusätzliche Einspeisung bringt einen zusätzlichen Energie- und Kühlwasserverbrauch mit sich. Außerdem muß diese Rektifikation entsprechend größer dimensioniert werden, was bei bestehenden Anlagen unmöglich ist. Als zur Rektifikation nötigen Rückfluß ist der Rücklauf aus den desorbierenden Adsorbern ungeeignet, da dieser die Kopfkonzentration aufweisen müßte.

Die herkömmliche Ethanoladsorptionstechnik, die mit zwei Adsorbern arbeitet, muß schlagartig von Beladungsdruck auf Regenerationsdruck umschalten, da sonst zu wenig Zeit zur Regeneration im Unterdruck zur Verfügung stehen würde. Diese Druckstöße verbunden mit Mengenstößen erschweren jedoch das Betreiben vor- bzw. nachgeschalteter Apparate. Dieses Problem wurde seither mit Zwischenkondensation, flüssiger Pufferung und flüssiger konstanter Einspeisung in die Rektifikation auf Kosten von Energie, Kühlwasser und apparativem Mehraufwand umgangen.

Eine weitere Schwierigkeit der bereits eingesetzten Adsorber stellt die Aufarbeitung der beim Regenerieren der Adsorber anfallenden Rückläufe dar.

Aufgabe der Erfindung ist es, ein Verfahren der eingangs genannten Art zu schaffen, das im Kreislauf gefahren wirksamer und wirtschaftlicher arbeitet als die bekannten Systeme.

Diese Aufgabe wird durch ein kombiniertes Adsorptions-Rektifikationsverfahren der eingangs genannten Art dadurch gelöst, daß man den dampfförmigen Beschickungsstrom aus einem Verdampfer oder aus dem Kopf eines Verdampfers oder einer Rektifikationskolonne durch ein aus mindestens drei periodisch gesteuerten Festbettadsorbern bestehendes Adsorptionssystem leitet, dessen Adsorbentien vornehmlich die höher siedende Flüssigkeit adsorbiert, wobei man die Adsorptionsbetten der Festbettadsorber abwechselnd regeneriert bzw. belädt und durch indirekte Beheizung ständig über dem Taupunkt des zu trennenden Flussigkeitsgemisches fährt, und daß man das abgetrennte, leicht siedende reine Endprodukt in einem

2

Kondensator kondensiert.

Zweckmäßige Weiterbildungen des erfindungsgemäßen Verfahrens sind in den Unteransprüchen gekennzeichnet.

Ein weites Anwendungsgebiet findet das kombinierte Adsorptions-Rektifikationsverfahren bei der Ethanolverarbeitung. Der dampfförmige Ethanolstrom wird aus einer normalen Rektifikation oder eines Verdampfers durch ein System von mindestens drei Festbettadsorbern geschickt. Diese werden durch periodisch arbeitende Ventile gesteuert und von unten nach oben unter Ausgangsdruck der Rektifikationskolonne oder des Verdampfers beladen, langsam bei geschlossenem oberen Ventil nach unten entleert, bei Unterdruck mit geschlossenem oberen Ventil regeneriert, langsam bei geschlossenem unteren Ventil von oben mit Endprodukt eines anderen Adsorbers, der gerade beladen wird, auf Beladungsdruck gebracht und anschließend erneut beladen.

Ein solcher periodisch wiederkehrender Zyklus eines jeden Adsorbers steht im Gegensatz zur herkömmlichen Ethanol adsorptionstechnik, die mit zwei Adsorbern arbeitet. Der Einsatz von mindestens drei Adsorbern ermöglicht die Aufrechterhaltung konstanter dampfförmiger Stoffströme und erübrigt die kostenintensive Zwischenkondensation. Die Feststoffadsorber in den Adsorptionsbetten werden periodisch so ad- bzw. desorbiert, daß es zu keinen Schwankungen hinsichtlich der Menge und des Druckes im Beschikkungsstrom der im System integrierten Rektifikation kommt. Dadurch kann ein normales Rektifikationsverfahren mit dampfförmiger Einspeisung bzw. Ausspeisung der Rektifikationen durchgeführt werden. Somit ist es möglich, daß zu jedem Zeitpunkt des Ad-/Desorptionszyklusses mindestens ein Adsorber von Adsorptionsdruck auf Desorptionsdruck entspannt bzw. von Desorptionsdruck auf Adsorptionsdruck beladen wird.

Wie weiter oben erwähnt ist, bereitet die Aufarbeitung der beim Regenerieren der Festbettadsorber anfallenden Rückläufe Schwierigkeiten. Erfindungsgemäß wird dieses Problem durch den Einsatz einer Vakuumrektifikation gelöst, die dampfförmig gespeist wird. Dies wird durch die zyklische Schaltung der Festbettadsorber möglich. Dabei wird der Unterdruck so gewählt, daß die Aufarbeitungskolonne mit Abwärme aus der Kondensation des einen, unter Beladedruck stehenden Festbettadsorbers verlassenden reinen Ethanols betrieben werden kann. Bei dieser Aufarbeitung entstehen im Sumpf der Aufarbeitungskolonne reines Wasser, im Kopf flüssigunterkühlter Ethanol mit einer azeotropen Konzentration von etwa 95 Vol.-%. Dieses Kopfprodukt kann in die der Adsorption vorgeschalteten Rektifikation als Rückfluß eingespeist werden und stellt somit keine zusätzliche Störung in diesem auf konstante Ströme angewiesenen Prozeß dar.

Der Unterdruck in der Aufarbeitungskolonne wird zweckmäßig durch mindestens eine Lavalldampfstrahldüse aufrechterhalten. Diese Düsen sind so konzipiert, daß ihr Abdampf und dessen Taupunkt groß bzw. hoch genug sind, um die Festbettadsorber durch indirekte Beheizung auf der notwendigen Betriebstemperatur zu halten, und dadurch zu vermeiden, daß durch Taupunktunterschreitung eine Kondensation im Adsorber auftritt. Das mit Kopfprodukt der Aufarbeitungskolonne behaftete, hierbei im Heizmantel der Adsorber anfallende Kondensat wird anschließend in der Aufarbeitungskolonne getrennt.

Anstelle der Lavalldampfstrahldüsen kann zur Aufrechterhaltung des nötigen Unterdrucks in der Aufarbeitungskolonne ein Flüssigkeitsstrahler eingesetzt werden. Dieser wird mit flüssigem Kopfprodukt der Aufarbeitungskolonne betrieben und ist mit einer Pumpe im Kreislauf geschaltet, während die Festbettadsorber indirekt mit Frischdampf beheizt werden.

Der Treibdampf der Dampfstrahldüsen ist somit die einzige Fremdenergie, die der dem erfindungsgemäßen Verfahren zugrundeliegende Trennprozeß benötigt. Seine Menge hängt in erster Linie von der zur Verfügung stehenden Kühlwassertemperatur und dem zur Verfügung stehenden Druck des Frischdampfes ab. Auf jeden Fall liegt der Energiebedarf weit unter dem der zum Heizen einer Kolonne bzw. eines Adsorbersystems notwendig wäre. Die gesamte übrige Energie wird dem Prozeß im dampfförmigen Ausgangsprodukt zugeführt. Da dieses Ausgangsprodukt jedoch beim Rektifizieren ohnehin kondensiert werden muß, um als notwendiger Rückfluß eingesetzt zu werden, bringt das erfindungsgemäße Verfahren keine zusätzlichen Energiekosten mit sich, erspart vielmehr einen Teil der Rückflußerzeuger. Nur die Kühlwassertemperatur muß entsprechend dem in der Aufarbeitungskolonne herrschenden Unterdruck niedriger sein, was unter Umständen einen gewissen Mehraufwand bedeutet. In keinem Fall ist jedoch der Einsatz von Kältemaschinen erforderlich.

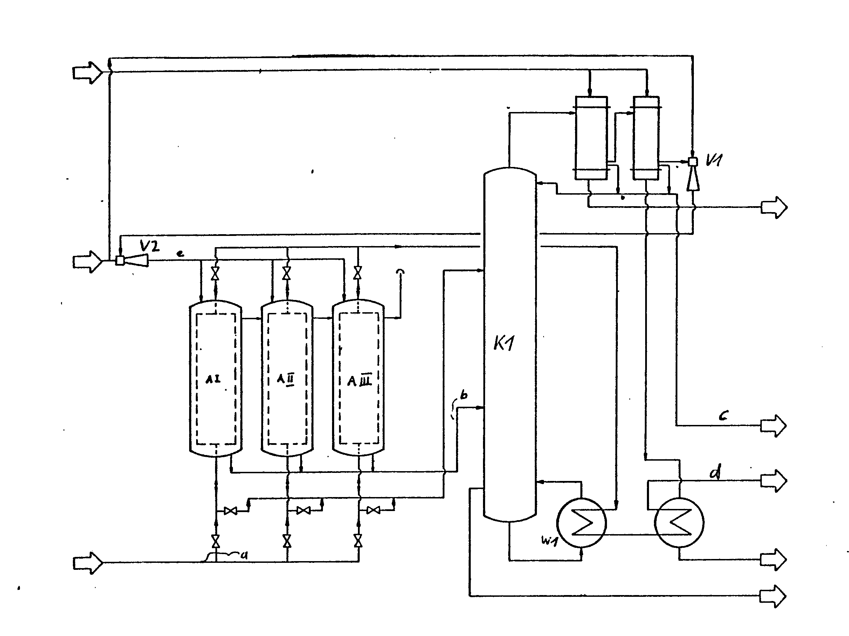

Das kombinierte Adsorptions-Rektifikationsverfahren wird anhand eines Beispiels näher erläutert. Die Bezugnahme auf eine in der beigefügten Zeichnung schematisch dargestellte Anlage dient zum leichteren Verständnis. Verarbeitet wurde ein Ethanol-Wassergemisch. Es versteht sich, daß auch andere Gemische, die aus unterschiedlich siedenden Flüssigkeiten bestehen, dem erfindungsgemäßen Trennverfahren unterworfen werden können.

Drei ventilgesteuerte Festbettadsorber AI, AII, AIII sind in Reihe geschaltet. Aus dem Boden dieser Festbettadsorber führen Leitungen zu einer Sammelleitung b, die in den unteren Bereich einer Aufarbei-

3

tungskolonne K1 mündet. Dampf wird über eine Leitung e in den Kopf der Festbettadsorber AI, AII, AIII geleitet. Ausgetragen wird durch die Leitung c Ethanol von 95 Vol.-% und durch die Leitung d Ethanol von 99,9 Vol.-%.

<u>Beispiel</u> bezogen auf 1.000 1 reinen Alkohol.

Alle Angaben in Liter beziehen sich auf flüssigen Zustand des entsprechenden Stoffstromes.

1.400 l Ethanol-Wassergemisch mit einer azeotropen Konzentration von 95 Vol.-% in Dampfform wurden durch Leitung a in den Adsorber AI geleitet, der bei leichtem Überdruck (1,2 bar (abs)) entsprechend dem Druck am Kopf der vorgeschalteten Kolonne bzw. des Verdampfers beladen wurde, dort von Wasser getrennt, wobei ein Teil des Ethanols ebenfalls im Festbettadsorber verblieb. Es verließen 1.000 1 Ethanol mit 99,9 Vol.-% den Adsorber, wurden im Sumpfkocher der Aufarbeitungskolonne K1 kondensiert und aus dem System durch die Leitung d ausgetragen.

Die übrigen 400 l wurden bei der Regeneration der Adsorber beim Regenerationsdruck von ca. 0,2 bar (abs) in die ebenfalls unter 0,2 bar (abs) betriebene Aufarbeitungskolonne K1 dampfförmig durch die Leitung b geleitet. Der Regenerationsdruck hängt unter anderem von der Kühlwassertemperatur, dem Dampfdruck des Treibdampfes der Lavalldampfstrahldüsen ab, und bestimmt das Volumen der Adsorber. In dieser Aufarbeitungskolonne K1 wurden diese 400 l in 347 l 95% Ethanol-Wassergemisch und 53 l reines Wasser getrennt.

Zum Aufrechterhalten des Regenerationsdruckes im desorbierenden Festbettadsorber bzw. der Aufarbeitungskolonne K1 und zur Heizung der Festbettadsorber wurden insgesamt rund 147 kg Dampf benötigt. Dieser Dampf wurde in der Lavalldüse verunreinigt, im Heizmantel der Festbettadsorber kondensiert und flüssig überhitzt in die Aufarbeitungskolonne K1 eingespeist und dort in seine Bestandteile getrennt.

Wie das Beispiel zeigt, wurden nur rund 0,15 kg Dampf zur Erzeugung von 1 l reinem Ethanol aus einem azeotropen Ethanol-Wassergemisches benötigt.

| Stoffbilanz | | |
|---|---|---|
| | Eintrag | Austrag |
| Ethanol 95 V.% | 1.400 l | 347 l |
| Ethanol 99,9 V.% | - | 1.000 l |
| Wasser | - | 53 l |
| Dampf | 150 kg | - |
| Kondensat | - | 150 kg |

**Ansprüche**

1. Kombiniertes Adsorptions-Rektifikationsverfahren zur Trennung eines aus unterschiedlich siedenden Flüssigkeiten bestehenden Flüssigkeitsgemisches, in eine höher siedende Flüssigkeit, insbesondere Wasser, und in einer tiefer siedende, insbesondere organische Flüssigkeit mit einer Anfangskonzentration von mehr als 80 Vol.-% durch Druckwechsel-Adsorption und Rektifikation, **dadurch gekennzeichnet**, daß man den dampfförmigen Beschickungsstrom aus einem Verdampfer oder aus dem Kopf eines Verdampfers oder einer Rektifikationskolonne durch ein aus mindestens drei periodisch gesteuerten Festbettadsorbern (AI, AII, AIII) bestehendes Adsorptionssystem leitet, dessen Adsorbentien vornehmlich die höher siedende Flüssigkeit adsorbieren, wobei man die Adsorptionsbetten der Festbettadsorber abwechselnd regeneriert bzw. belädt und durch indirekte Beheizung ständig über dem Taupunkt des zu trennenden Flüssigkeitsgemisches führt, und daß man das abgetrennte, leicht siedende reine Endprodukt in einem Kondensator (W1) kondensiert.

2. Verfahren nach Anspruch 1, dadurch gekennzeichnet, daß man die Festbettadsorber (AI, AII, AIII) durch Drucksenkung regeneriert.

3. Verfahren nach Anspruch 1, dadurch gekennzeichnet, daß man das bei der periodischen Regeneration der mit der adsorbierten Flüssigkeit beladenen Festbettadsorber (AI, AII, AIII) anfallende dampfförmige Flüssigkeitsgemisch in eine unter Unterdruck betriebene Aufarbeitungskolonne (K1) leitet und den Regene-

EP 0 357 832 A1

rationsstrom in eine flüssigunterkühlte Flüssigkeit, deren Konzentration dem Beschickungsstrom der Festbettadsorber (AI, AII, AIII) entspricht, und in eine reine, schwerer siedende Komponente trennt.

4. Verfahren nach Anspruch 1 und 3, dadurch gekennzeichnet, daß man das am Kopf der Aufarbeitungskolonne (K1) gebildete unterkühlte Flüssigkeitsgemisch zu dem der Adsorption vorgeschalteten Verdampfer bzw. der Rektifikation zurückführt und hier gegebenenfalls als Rückfluß verwendet.

5. Verfahren nach Anspruch 1, dadurch gekennzeichnet, daß man ein Ethanol/Wassergemisch verarbeitet und als dampfförmiges Flüssigkeitsgemisch das Kopfprodukt einer Ethanolrektifikation oder eines Ethanolverdampfers verwendet.

6. Verfahren nach Anspruch 1, dadurch gekennzeichnet, daß die Festbettadsorber (AI, AII, AIII) periodisch so adsorbiert bzw. desorbiert werden, daß keine Schwankungen hinsichtlich der Menge und Druck im Beschickungsstrom (a, b) der im System integrierten Rektifikationen auftreten, wobei mindestens ein Adsorberbett unter Adsorptionsdruck beladen und mindestens ein Adsorberbett und Desorptionsdruck regeneriert wird.

7. Verfahren nach Ansprüchen 1, 2 und 3, dadurch gekennzeichnet, daß zur Aufarbeitung des Rektifikationsstromes der Unterdruck der Rektifikation zur Abtrennung der schwerer siedenden Komponente so gewählt wird, daß die Kondensationswärme der abgetrennten leichter siedenden Komponente als Sumpfheizung in der Aufarbeitungskolonne (K1) verwendet werden kann.

8. Verfahren nach Anspruch 3, dadurch gekennzeichnet, daß zur Aufrechterhaltung des Unterdrucks in der Aufarbeitungskolonne (K1) mindestens eine Lavalldampfstrahldüse verwendet wird.

9. Verfahren nach Anspruch 3, dadurch gekennzeichnet, daß zur Aufrechterhaltung des Unterdrucks in der Aufarbeitungskolonne. (K1) ein Flüssigkeitsstrahler eingesetzt wird, der mit flüssigem Kopfprodukt der Aufarbeitungskolonne (K1) betrieben wird und mit einer Pumpe im Kreislauf geschaltet ist, während die Festbettadsorber (AI, AII, AIII) indirekt mit Frischdampf beheizt werden.

10. Verfahren nach Anspruch 1, dadurch gekennzeichnet, daß man ein 95 Vol.-% Ethanol-Wassergemisch einer adsorptiven Azeotropentrennung in Kombination mit an sich bekannter Rektifikation unterwirft.

5

EP 0 357 832 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.3) |
|---|---|---|---|
| X | EP-A-0 092 208 (UNION CARBIDE) <br> * Figur; Seite 19, Zeile 21 - Seite 24, Zeile 7 * <br> --- | 1-5,10 | C 07 C 29/76 <br> B 01 D 3/14 <br> C 12 F 1/08 |
| X | US-A-4 351 732 (J.D. PSARAS et al.) <br> * Anspruch 7; Figur 6 * <br> --- | 1,3,10 | |
| X | WO-A-8 603 686 (UNION CARBIDE) <br> * Ansprüche 1-5 * <br> --- | 1 | |
| A | EP-A-0 158 754 (CALGON CARBON C.) <br> ----- | | |

|  |
|---|
| **RECHERCHIERTE SACHGEBIETE (Int. Cl.3)** |
| B 01 D 3/00 <br> B 01 D 53/00 <br> C 07 C 29/00 <br> C 07 C 31/00 <br> C 12 F 1/00 |

**Der vorliegende Recherchenbericht wurde für alle Patentansprüche erstellt**

| Recherchenort | Abschlußdatum der Recherche | Prüfer |
|---|---|---|
| BERLIN | 05-04-1989 | KESTEN W.G. |